# EUROPÄISCHE PATENTSCHRIFT

(11) **EP 4 252 608 B1**
(45) Veröffentlichungstag und Bekanntmachung des Hinweises auf die Patenterteilung: **02.12.2026**
(21) Anmeldenummer: 22166205.9
(22) Anmeldetag: 31.03.2022
(51) Int. Cl.: A47L 11/20, A47L 11/206

(54) **VORRICHTUNG ZUM REINIGEN EINES RAUMS IN EINER FAHRZEUGKABINE**
DEVICE FOR CLEANING A ROOM IN A VEHICLE CABIN
DISPOSITIF DE NETTOYAGE D'UN ESPACE DANS UNE CABINE DE VÉHICULE

(43) Veröffentlichungstag der Anmeldung: 04.10.2023
(73) Patentinhaber: Airbus Operations GmbH, 21129 Hamburg (DE)
(72) Erfinder: Muin, Andrew, 21129 Hamburg (DE); Sontag, Stephan, 21129 Hamburg (DE)
(74) Vertreter: Airbus Germany

(56) Entgegenhaltungen:
- CN-A- 108 433 643
- DE-A1- 102007 029 258
- US-A1- 2005 005 389
- US-A1- 2014 259 516
- US-A1- 2021 369 901

## Beschreibung

### Technisches Gebiet

Die vorliegende Beschreibung betrifft eine Vorrichtung zum Reinigen eines Raums in einer Fahrzeugkabine sowie ein Luftfahrzeug mit einem Raum mit mehreren Wandungen und einer derartigen Vorrichtung zum Reinigen des Raums.

### Technischer Hintergrund

Die Reinigung von Fußböden und anderen Bereichen von Waschräumen in Luftfahrzeugen ist aufgrund der üblicherweise sehr kompakten Abmessungen schwierig durchzuführen. Da die Anzahl an Besatzungsmitgliedern eines Luftfahrzeugs überdies begrenzt ist und vielfältige andere Aufgaben zu erledigen sind, wird eine Reinigung während eines Flugs normalerweise nicht durchgeführt. Es sind dauerhaft und fest installierte Vorrichtungen zum Reinigen von Toiletten sowie an Fußbodenrändern installierte Saugvorrichtungen zum frequentierten Einsaugen von Luft zum Aufnehmen von losen Schmutzpartikeln bekannt. Sollten Fehlfunktionen oder Defekte eine Reparatur erfordern ist hierfür erforderlich, das Luftfahrzeug temporär am Boden zu lassen. Es kann gelegentlich vorkommen, dass zu stark verschmutzte Waschräume der Einfachheit halber während eines Fluges verschlossen werden, sodass sie temporär nicht nutzbar sind.

DE 10 2007 029258 A1 betrifft ein Bodenreinigungsgerät mit einem Bürstenkopf, in dem eine Bürstenwalze drehbar gelagert ist und der mindestens eine neben der Bürstenwalze angeordnete Saugdüse aufweist, und mit einem schwenkbar am Bürstenkopf gehaltenen Stiel, an dem ein Saugaggregat angeordnet ist sowie ein Reinigungsflüssigkeitsbehälter für Reinigungsflüssigkeit, die auf die zu reinigende Bodenfläche aufbringbar ist, und ein Schmutzflüssigkeitsbehälter zur Aufnahme von mittels des Saugaggregates von der Bodenfläche aufgesaugter Schmutzflüssigkeit, wobei der Reinigungsflüssigkeitsbehälter und der Schmutzflüssigkeitsbehälter gemeinsam eine mit dem Stiel lösbar verbundene Behälterbaugruppe ausbilden. Um das Bodenreinigungsgerät derart weiterzubilden, dass es eine einfachere Handhabung aufweist, wird vorgeschlagen, dass der Reinigungsflüssigkeitsbehälter mit dem Schmutzflüssigkeitsbehälter lösbar verbindbar ist. Eine UV-Leuchte ist nicht vorgesehen.

### Beschreibung

Es ist eine Aufgabe der Erfindung, eine Vorrichtung zum Reinigen eines Waschraums in einem Luftfahrzeug vorzuschlagen, welche zuverlässig und möglichst automatisch betreibbar ist und eine Aufrechterhaltung der Funktion des Waschraums gewährleisten kann.

Die Aufgabe wird gelöst durch eine Vorrichtung zum Reinigen eines Raums in einer Fahrzeugkabine mit den Merkmalen des Anspruchs 1. Vorteilhafte Ausführungsformen und Weiterbildungen sind den Unteransprüchen und der nachfolgenden Beschreibung zu entnehmen.

Es wird eine Vorrichtung zum Reinigen eines Raums in einer Fahrzeugkabine vorgeschlagen, die Vorrichtung aufweisend ein Gehäuse mit einer Oberseite und einer Unterseite, mehrere an der Unterseite angeordnete Räder, mindestens eine an der Unterseite angeordnete Bürsteneinheit, mindestens eine Saugeinheit, eine Fluidzufuhreinheit, eine Antriebseinheit, eine Steuereinheit, und mindestens eine an der Oberseite des Gehäuses bewegbar angeordnete UV-Leuchte zum Sterilisieren von Oberflächen, wobei die Antriebseinheit mit mindestens einem der Räder zum Bewegen der Vorrichtung auf einem Fußboden des Raums gekoppelt ist, wobei die Fluidzufuhreinheit mindestens eine Abgabedüse aufweist, die auf den Fußboden und/oder zumindest eine der mindestens einen Bürsteneinheit gerichtet ist, wobei die mindestens eine Saugeinheit mindestens eine Saugdüse aufweist, die auf den Fußboden und/oder zumindest eine der mindestens einen Bürsteneinheit gerichtet ist und dazu ausgebildet ist, Feststoffe und/oder Flüssigkeiten aufzusaugen, wobei die mindestens eine Bürsteneinheit dazu ausgebildet ist, durch eine rotierende Bewegung den Fußboden zu reinigen, und wobei die Steuereinheit mit der Antriebseinheit, der mindestens einen Saugeinheit, und der Fluidzufuhreinheit gekoppelt ist und dazu ausgebildet ist, die Vorrichtung durch Ansteuerung der Antriebseinheit auf dem Fußboden zu verfahren und dabei durch Ansteuerung der mindestens Bürsteneinheit, der Fluidzufuhreinheit und der Saugeinheit den Fußboden zu reinigen.

Die erfindungsgemäße Vorrichtung ist bewegbar, und kann zum Reinigen in den Raum gebracht werden. Durch Räder an der Unterseite kann die Vorrichtung auf dem Fußboden aufstehen und bewegt werden. Die Räder sind an der Unterseite des Gehäuses angeordnet und ragen bevorzugt nicht über eine Grundrisskontur des Gehäuses hinaus. Es ist vorstellbar, dass die Vorrichtung drei oder vier Räder aufweist, um einen sicheren Stand zu gewährleisten. Zum Bewegen der Vorrichtung von außerhalb des Raums in den Raum hinein könnte ein Griff vorgesehen sein, der von einem Benutzer gegriffen wird, um die Vorrichtung in den Raum zu führen.

Die Antriebseinheit befähigt die Vorrichtung dazu, sich automatisch innerhalb des Raums auf dem Fußboden fortzubewegen. Sie könnte etwa einen oder mehrere Elektromotoren umfassen, welche von einem elektrischen Energiespeicher mit einer elektrischen Spannung versorgt werden. Bevorzugt ist die Kombination aus den Rädern und der Antriebseinheit dazu ausgebildet, dass die Vorrichtung lenken kann und folglich entlang einer vorbestimmten oder während des Gebrauchs automatisch ermittelten Bahn innerhalb des Raums fahren kann. In beengten Waschräumen kann die Bahn relativ kurz sein und sich von einer Tür bis zu einer Toiletteneinheit erstrecken. Es ist vorstellbar, dass sich die Vorrichtung durch gegenläufiges Antreiben von Rädern auch um eine Hochachse drehen kann, um zusätzlich zu einer ersten Bewegungsrichtung eine zweite, einen Winkel von etwa 90° oder weniger, hierzu einschließende Bewegungsrichtung einzunehmen. Durch den Antrieb der Räder könnte die Vorrichtung auch dazu befähigt sein, automatisch von einem Stellplatz in den Raum zu fahren.

Zur effektiven Reinigung des Fußbodens ist die mindestens eine Bürsteneinheit vorgesehen. Diese weist eine rotierende Bürste auf, welche mit dem Fußboden in Kontakt bringbar ist, um dort Partikel zu bewegen und/oder aufzunehmen. Die mindestens eine Bürsteneinheit kann auf mehrere unterschiedliche Arten ausgebildet sein. Grundsätzlich bietet es sich an, tellerartige Bürsten und/oder walzenartige Bürsten einzusetzen, wobei die Rotationsachse einer tellerartigen Bürste im Wesentlichen vertikal zu dem Fußboden verlaufen kann, die Rotationsachse einer walzenartigen Bürste im Wesentlichen parallel zu dem Fußboden. Selbstverständlich können mehrere derartige Bürsteneinheiten in Kombination miteinander Verwendung finden.

Die Fluidabgabeeinheit könnte insbesondere mit einem Fluidtank verbunden sein, in dem ein Reinigungsmittel oder Wasser mit einem Reinigungsmittelzusatz enthalten ist. Das Fluid kann über eine Pumpe oder schwerkraftgetrieben einer oder mehreren Abgabedüsen zugeführt werden. Diese sprühen das Fluid direkt auf den Fußboden und/oder auf zumindest eine der mindestens einen Bürsteneinheit. Durch das Aufsprühen auf den Fußboden könnte eine dort befindliche Verschmutzung angelöst werden, um in einem nachfolgenden Arbeitsgang von der mindestens einen Bürsteneinheit aufgenommen zu werden. Es wäre prinzipiell denkbar, auch eine zweite Fluidzufuhreinheit einzusetzen, die mit einem weiteren Fluidtank verbunden ist, in dem klares Wasser enthalten ist. Damit könnte beispielsweise ein Nachspülen erfolgen.

Eine erste Saugdüse könnte strategisch so platziert sein, dass die mindestens eine Bürsteneinheit auf dem Boden befindliche Partikel in Richtung der Saugdüse bewegt, sodass sie dort unmittelbar aufgenommen werden können. Wird die Vorrichtung beim Bürsten verfahren, wäre denkbar, dass die mindestens eine Bürsteneinheit großräumig Schmutzpartikel zu einer schmalen Spur oder eine Anhäufung von Schmutzpartikeln zusammenkehrt, die im vorgesehenen Fahrweg der Vorrichtung und in einem Bereich angeordnet ist, der durch die erste Saugdüse überstrichen wird.

Die mindestens eine Saugeinheit ist mit einem Behälter gekoppelt, in dem aufgesaugte Schmutzpartikel gesammelt werden. Es könnte sich hierbei um einen beutellosen oder mit einem Beutel ausgestatteten Behälter handeln, die regelmäßig entleert werden können.

Es ist weiterhin denkbar, eine zweite Saugdüse einzusetzen, die mit dem Fußboden in Flächenkontakt gerät und einen darauf liegenden Flüssigkeitsfilm aufsaugen kann, um den Fußboden weitgehend zu trocknen. Die zweite Saugdüse liefert dann aufgesaugte Flüssigkeit in einen Abwassertank.

Die Steuereinheit ist mit den aktiven Komponenten verbunden und könnte dazu ausgebildet sein, einen vorbestimmten Verfahrweg abzurufen und die aktiven Komponenten derart anzusteuern, dass entlang dieses vorgesehenen Verfahrwegs eine Reinigung erfolgt. Prinzipiell ist auch denkbar, dass die Vorrichtung mit einem oder mehreren Sensoren ausgestattet ist, die charakteristische Merkmale des betreffenden Raums aufnehmen können, um anschließend einen Verfahrweg zu planen, welcher dann von der Vorrichtung abgefahren wird. Die Steuereinheit könnte in einem etwas einfacheren Fall jedoch auch dazu eingerichtet sein, stets einen spiralförmigen, Zickzack förmigen oder chaotischen Verfahrweg abzufahren, wobei gleichzeitig durch die Sensoren erfasst werden kann, ob eine Kollision mit einem Einbaugegenstand erfolgen könnte.

Es ist vorgesehen, dass die Vorrichtung bevorzugt vollautomatisch den Reinigungsvorgang durchführt. Hierzu könnte ein Benutzer die Vorrichtung über einen Griff oder dergleichen in den betreffenden Raum führen. Damit die Räder sich hierzu drehen lassen, könnte ein Freilauf an dem jeweils angetriebenen Rad vorgesehen sein. Die Vorrichtung könnte weiterhin einen Taster, Schalter oder einen anderen Impulsgeber aufweisen, durch den der Benutzer den Reinigungsvorgang initiieren kann. Die Steuereinheit ist mit diesem Impulsgeber verbunden und könnte nach Erkennen des Betätigens unmittelbar mit dem Reinigungsvorgang starten.

Der besondere Vorteil der erfindungsgemäßen Vorrichtung liegt in der Kombination des Bürstens, des Saugens und des Verwendens einer Reinigungsflüssigkeit, um in einem sehr beengten Waschraum eines Luftfahrzeugs eine bedarfsweise gründliche Reinigung durchführen zu können, sodass der Raum betriebsbereit ist. Hiermit könnte sogar die Anzahl der Waschräume in einem Luftfahrzeug reduzierbar sein, da durch die bedarfsweise Reinigung sämtliche Waschräume einsatzbereit bleiben. Es kann vorgesehen sein, die Vorrichtung derart dimensioniert wird, dass sie bei Nichtbenutzung in einem bereits vorhandenen Stellplatz abgestellt werden könnte, beispielsweise in einem Trolley-Stellplatz. Da sich eine Bordküche oftmals in der Nähe von Bordtoiletten befindet, ist dies besonders günstig und die Vorrichtung muss nur eine kurze Strecke bewegt werden.

Erfindungsgemäß weist die Vorrichtung ferner mindestens eine an der Oberseite des Gehäuses bewegbar angeordnete UV-Leuchte zum Sterilisieren von Oberflächen auf. Die UV-Leuchte kann zum Erreichen von zu sterilisierenden Oberflächen bewegt werden. Das Bewegen kann beispielsweise manuell vor dem Einsetzen der Vorrichtung erfolgen. Da, wie vorangehen dargelegt, die Vorrichtung besonders bevorzugt bei Nichtbenutzung in einem Stellplatz verstaut wird, kann die mindestens eine UV-Leuchte eingeklagt, eingefahren oder auf andere Art kompakter werden. Ein Benutzer könnte nach Einbringen der Vorrichtung in den zu reinigenden Raum die mindestens eine UV-Leuchte ausklappen oder ausfahren. Andererseits ist auch denkbar, dass die mindestens eine UV-Leuchte durch einen Akt hoher Tor bewegt werden kann, um aus der eingefahrenen Position in eine ausgefahrene und passend platzierte Lage gebracht wird.

In einer vorteilhaften Ausführungsform ist die mindestens eine UV-Leuchte teleskopierbar und/oder schwenkbar angeordnet, sodass eine Oberfläche eines Waschtischs und/oder ein Türgriff damit beleuchtbar ist. Die UV-Leuchte könnte ein Leuchtstab sein und etwa mehrere LEDs oder andere Leuchtmittel umfassen, die stabförmig angeordnet sind. Das Licht wird dann von einer linienförmigen Lichtquelle abgegeben. Der Waschtisch könnte eine Höhe aufweisen, die in einem Bereich von 0,8 m bis 1 m liegt. Die UV-Leuchte könnte so ausgestaltet sein, dass die linienförmige Lichtquelle waagerecht und parallel zu dem Waschtisch ausgerichtet wird, um so eine Desinfektion der Oberfläche auszuführen. Die UV-Leuchte könnte, um die Armaturen und rückwärtige Bereiche des Waschtischs ausreichend auszuleuchten, um einen Winkel von etwa 10° bis 30° zu dem Waschtisch bzw. dem Fußboden ausgerichtet werden, wobei die UV-Leuchte an der Vorrichtung ihre niedrigste Höhe aufweist und sich von der Vorrichtung weg über den Waschtisch mit einem zunehmenden Abstand von dem Waschtisch erstreckt. Zum Beleuchten von Türgriffen ist denkbar, eine separate UV-Leuchte zu verwenden, wobei die linienförmige Lichtquelle senkrecht zu dem Fußboden ausgerichtet sein könnte. Es sind auch andere Lichtquellen denkbar, die nicht linienförmig sind, sondern flächig oder punktförmig sind. Diese sind dann so zu platzieren, dass eine ausreichende Ausleuchtung der zu desinfizierenden Bereiche erfolgen kann. Die Vorrichtung kann sich bei der Desinfektion durch UV-Licht bewegen, sodass die Lichtquelle lediglich einen Bereich der zu desinfizierenden Fläche erreichen muss und die Vorrichtung durch Bewegung die Lichtquelle über die betreffende Fläche überstreichen lässt. Weiterhin ist denkbar, eine Toilette und/oder einen Fußboden durch die mindestens eine UV-Leuchte zu desinfizieren.

In einer vorteilhaften Ausführungsform weist die Vorrichtung ferner eine Luftreinigungseinheit auf, die dazu ausgebildet ist, Luft aus dem zu reinigenden Raum einzusaugen und gereinigt wieder abzugeben. Die Luftreinigungseinheit ist insbesondere dafür vorgesehen, unangenehme Gerüche und Schwebstoffe aus der Luft zu entfernen und damit eine Reinigung durchzuführen. Die Luftreinigungseinheit kann beispielsweise einen oder mehrere Filter umfassen, unter anderem etwa einen Aktivkohlefilter.

In einer vorteilhaften Ausführungsform weist die Luftreinigungseinheit mindestens einen Luftkanal auf, durch den die eingesaugte Luft strömt und mittels UV-Strahlen gereinigt wird. Die Luftreinigungseinheit weist folglich ebenso eine UV-Leuchte auf, welche etwaige, in der Luft enthaltene Krankheitserreger, Bakterien, Viren oder andere Keime abtötet bzw. in ihrer Wirkung reduziert. Eingesaugte Luft könnte in mehrere transparente Luftkanäle geleitet werden, die sich von einem Lufteingang zu einem Luftausgang erstrecken. Die Luftkanäle können etwa in einer Matrixform oder einer ähnlichen regelmäßigen oder unregelmäßigen Anordnung vorliegen. Eine oder mehrere UV-Leuchten können dann diese Luftkanäle mit UV-Licht beaufschlagen und damit eine Keimreduktion durchführen.

In einer vorteilhaften Ausführungsform weist die mindestens eine Bürsteneinheit mehrere Eckbürsteneinheiten auf, die an Ecken des Gehäuses angeordnet sind und jeweils um eine erste Drehachse rotierbar sind, die zu dem Fußboden im Wesentlichen vertikal verläuft. Eine Eckbürsteneinheit ist bevorzugt an einer Ecke des Gehäuses angeordnet und ist in der Lage, eine Eckfläche des Fußbodens zu reinigen. Die Eckbürsteneinheiten sind bevorzugt paarweise gegenläufig angetrieben oder antreibbar. Die Eckbürsteneinheiten fegen damit Partikel von einem Fußboden in Richtung einer vorgesehenen Fahrspur, entlang derer die Vorrichtung verfahren wird. Dann kann die Saugeinheit die örtlich konzentrierten Partikel aufsaugen. Die Eckbürsteneinheiten weisen jeweils eine tellerförmige Bürste auf, die um eine zu dem Fußboden vertikale Achse drehbar ist. Sie könnte einen flachen Ring oder eine flache Scheibe mit quer daran angeordneten Borsten umfassen. Die Eckbürsteneinheiten könnten mit einem leichten Druck auf den Fußboden gedrückt werden. Es ist denkbar, dass jede Eckbürsteneinheit einen eigenen Antrieb aufweist, der beispielsweise einen Getriebemotor umfasst, der direkt mit der Bürste gekoppelt ist.

In einer vorteilhaften Ausführungsform ist mindestens eine Eckbürsteneinheit relativ zu dem Gehäuse bewegbar, sodass sie einen variablen Abstand und/oder eine variable Ausrichtung zu dem Gehäuse einnehmen kann. Dies ermöglicht zum einen eine besonders kompakte Gestaltung der Vorrichtung, die das Verstauen in einem Stellplatz verbessert. Zum anderen erlaubt das Bewegen der Eckbürsteneinheiten, dass auch schwierig zugängliche Eckbereiche des Fußbodens mit einer Bürste überstrichen werden können. Beispielsweise könnten eine oder mehrere Eckbürsteneinheiten an jeweils einem verschwenkbaren Arm angeordnet sein, der bedarfsweise um eine zu dem Fußboden vertikale Achse herum verschwenkt wird, um die betreffende Bürste von dem Gehäuse zu beabstanden. Weiterhin ist denkbar, dass eine Teleskopieranordnung oder ähnliches vorgesehen ist, um die Eckbürsteneinheit radial nach außen zu verschieben.

In einer vorteilhaften Ausführungsform umfasst die mindestens eine Bürsteneinheit vier Eckbürsteneinheiten, die an vier Ecken der Unterseite des Gehäuses angeordnet sind. Hiermit wird eine zusätzliche Stabilisierung der Lage des Gehäuses erreicht, insbesondere wenn alle Eckbürsteneinheiten radial nach außen bewegbar sind. Die Reinigungswirkung wird zudem deutlich beschleunigt, da gleichzeitig vier Bürsten den Fußboden behandeln.

In einer vorteilhaften Ausführungsform weist die mindestens eine Bürsteneinheit eine Walzenbürsteneinheit auf, die um eine zweite Drehachse rotierbar ist, die im Wesentlichen parallel zu dem Fußboden ausgerichtet ist. Die Walzenbürsteneinheit könnte sich im Wesentlichen über die gesamte Breite der Unterseite des Gehäuses erstrecken und insbesondere in einer Richtung rotieren, durch die Schmutzpartikel der Saugdüse zugeführt werden. Die durch die Eckbürsteneinheiten auf einen begrenzten Pfad konzentrierten Schmutzpartikel können folglich leicht aufgesaugt werden. Durch die große Breite der Walzenbürsteneinheit erfolgt zudem eine gründliche Reinigung des Fußbodens.

In einer vorteilhaften Ausführungsform ist die Walzenbürsteneinheit zwischen der mindestens einen Abgabedüse und der mindestens einen Saugdüse angeordnet. Dies erlaubt das direkte Zuführen von Schmutzpartikeln an die Saugdüse. Dabei kann die mindestens eine Saugdüse länglich ausgebildet sein und beispielsweise parallel zu der zweiten Drehachse verlaufen. Die Saugdüse könnte eine Länge aufweisen, die der Breite der Walzenbürsteneinheit entspricht oder sie leicht übersteigt. Die Saugdüse könnte zudem segmentiert sein und mehrere einzelne Düsen umfassen, die zusammen die genannte Form ergeben. Die Abgabedüse könnte auf ähnliche Weise gestaltet sein wie die Saugdüse.

In einer vorteilhaften Ausführungsform ist die Walzenbürsteneinheit mittig an der Unterseite angeordnet. Die Kontaktfläche der Walzenbürsteneinheit mit dem Fußboden liegt folglich im Wesentlichen mittig zwischen einer Vorderseite und einer Rückseite des Gehäuses. Für die Abgabedüse und die Saugdüse ist dann ein im Wesentlichen identischer Bauraum vorhanden und die Verfahr-Richtung der Vorrichtung kann je nach Anforderung gewechselt werden, sodass unabhängig von der Verfahr-Richtung ein identisches Reinigungsergebnis erreicht wird.

In einer vorteilhaften Ausführungsform weist die Vorrichtung ferner eine Warmluftabgabeeinheit mit mindestens einer Warmluftdüse auf, die an der Unterseite angeordnet und auf den Fußboden gerichtet ist, um warme Luft zum Trocknen des Fußbodens abzugeben. Die Warmluftabgabeeinheit könnte eine elektrische Heizvorrichtung aufweisen, mit der einströmende Luft aufgeheizt und dem Fußboden zugeführt wird. Die Warmluftabgabeeinheit könnte einen Lüfter, ein Gebläse oder einen Verdichter umfassen, welcher mit einer Luftaufnahmeöffnung gekoppelt ist, die beispielsweise an einer Seitenfläche des Gehäuses angeordnet ist. Durch die Warmluftabgabeeinheit kann folglich der Fußboden nach der Feuchtreinigung getrocknet werden und der betreffende, zu reinigende Raum kann unmittelbar wieder genutzt werden.

In einer vorteilhaften Ausführungsform weist das Gehäuse eine Größe auf, die im Wesentlichen der Größe eines Half-Size-Trolley für Luftfahrzeugkabinen entspricht. Der Trolley könnte etwa dem "Atlas" Standard entsprechen und eine Länge von etwa 0,4 m, eine Breite von etwa 0,3 m und eine Höhe von etwa 1 m aufweisen. Damit kann sichergestellt werden, dass die erfindungsgemäße Vorrichtung ohne Modifikationen in einem üblichen Trolley-Stellplatz untergebracht werden kann.

Die Erfindung betrifft ferner ein Luftfahrzeug, aufweisend einen Raum mit mehreren Wandungen und einem Fußboden, sowie eine Vorrichtung nach der vorhergehenden Beschreibung.

In einer vorteilhaften Ausführungsform ist der Raum ein Waschraum mit einer Toilette und/oder einem Waschtisch, wobei der Raum ein Waschraum mit einer Toilette und/oder einem Waschtisch ist, und wobei das Luftfahrzeug einen Trolley-Stellplatz aufweist, in dem die Vorrichtung abstellbar ist.

### Kurze Beschreibung der Figuren

Nachfolgend wird anhand der beigefügten Zeichnungen näher auf Ausführungsbeispiele eingegangen. Die Darstellungen sind schematisch und nicht maßstabsgetreu. Gleiche Bezugszeichen beziehen sich auf gleiche oder ähnliche Elemente. Es zeigen:
- Fig. 1: eine schematische Darstellung einer Vorrichtung in einer Seitenansicht mit einem Waschraum und einem Benutzer.
- Fig. 2: eine schematische Darstellung einer Vorrichtung mit einer Luftreinigungseinheit.
- Fig. 3: eine schematische Schnittdarstellung von der Seite.
- Fig. 4: eine schematische Schnittdarstellung von vorne.
- Fig. 5: eine schematische Ansicht von der Unterseite in kompaktierter Stellung.
- Fig. 6: eine schematische Ansicht von der Unterseite in Benutzungsstellung.
- Fig. 7: eine schematische Ansicht der Vorrichtung schräg von der Unterseite.
- Fig. 8: eine Darstellung zum Größenvergleich der Vorrichtung mit einem Benutzer.
- Fig. 9: die Vorrichtung in einem Waschraum.
- Fig. 10: einen Stellplatz für die Vorrichtung.
- Fig. 11: ein Luftfahrzeug.

### Detaillierte Beschreibung von Ausführungsbeispielen

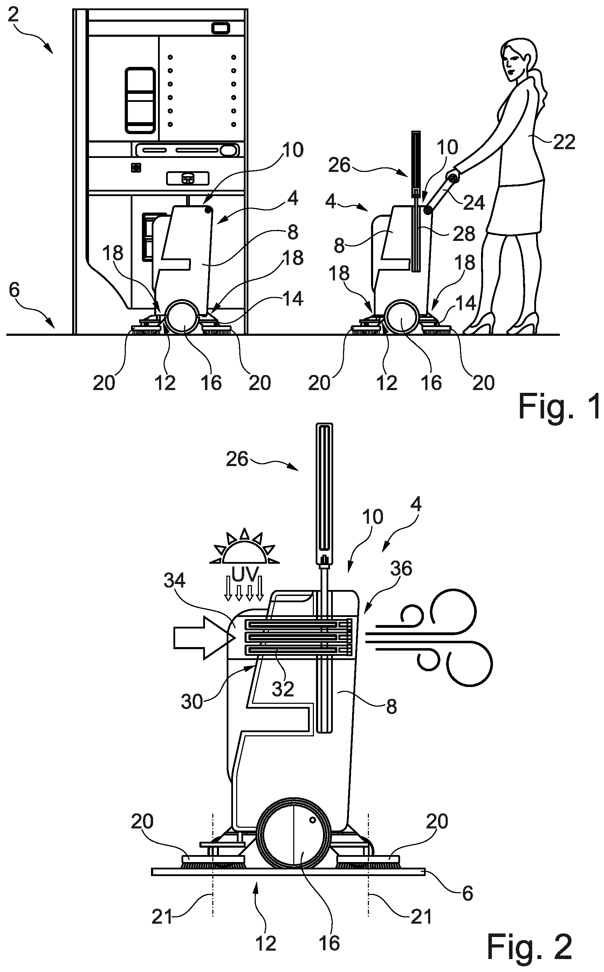

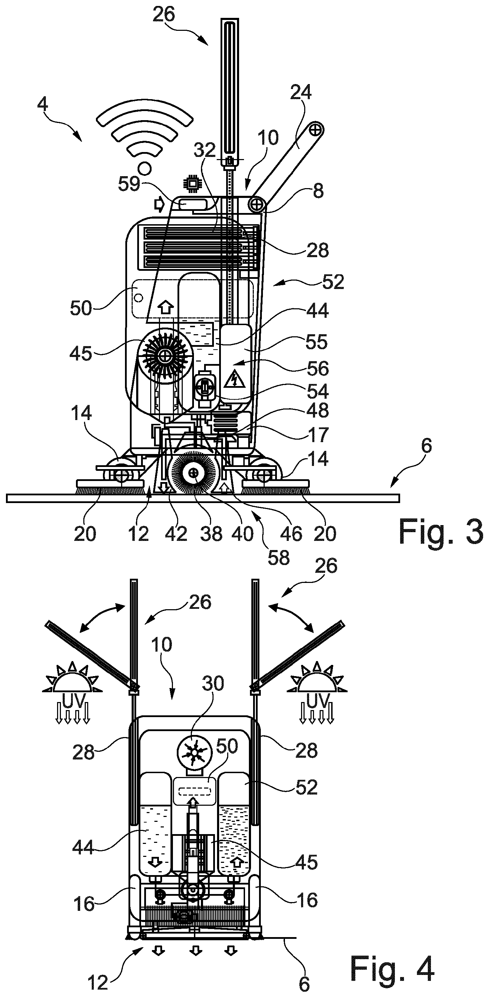

Fig. 1 zeigt einen Waschraum 2 in einer Schnittdarstellung. Hier ist eine Vorrichtung 4 zum Reinigen des Waschraums 2 angeordnet und steht dort auf einem Fußboden 6. Die Vorrichtung weist ein Gehäuse 8 mit einer Oberseite 10 und einer Unterseite 12 auf. An der Unterseite 12 sind mehrere Räder 14 und 16 angeordnet. Mindestens eines der Räder 14 und 16 ist elektrisch angetrieben, etwa durch eine in Fig. 3 ersichtliche Antriebseinheit 17, sodass sich die Vorrichtung 4 selbstständig bewegen kann. An Ecken 18 des Gehäuses 8 sind Eckbürsteneinheiten 20 angeordnet, die auf dem Fußboden 6 aufliegen und jeweils um eine erste Drehachse 21 rotierbar sind. Die erste Drehachse 21 kann vertikal zu dem Fußboden 6 verlaufen. Die Vorrichtung 4 dient der Reinigung des Fußbodens 6 des Waschraums 2 und kann, wie auf der rechten Seite von Fig. 1 dargestellt, von einem Benutzer 22 beispielsweise an einem Handgriff 24 in den Waschraum 2 hinein geführt werden. Dort kann die Vorrichtung 4 selbstständig einen Reinigungsvorgang ausführen. Die hierfür vorgesehenen einzelnen Komponenten werden anhand der weiter nachfolgenden Figuren erläutert.

In Fig. 1 ist weiterhin eine UV-Leuchte 26 dargestellt, die an der Oberseite 10 aus einem länglichen, bevorzugt vertikal ausgerichteten Hohlraum 28 teleskopierbar ist. Sie dient der Desinfektion von Flächen innerhalb des Waschraums 2. Beispielhaft ist die UV-Leuchte 26 länglich ausgebildet und gibt Licht aus einer linienförmigen Lichtquelle ab, die etwa mehrere, aneinander gereihte LEDs aufweisen kann.

Fig. 2 zeigt eine vergrößerte Seitenansicht der Vorrichtung 4. Hier ist schematisch eine Luftreinigungseinheit 30 dargestellt, in der eine Vielzahl von Luftkanälen 32 angeordnet sind. Diese können mittels UV-Strahlen behandelt werden, sodass darin enthaltene Keime abgetötet bzw. in ihrer Anzahl reduziert werden. Luft strömt durch einen Lufteingang 34 ein und strömt nach Passieren der Luftkanäle 32 aus einem Luftausgang 36 wieder aus. Eine UV-Lichtquelle könnte an den Kanälen 32 angeordnet werden oder durch die teleskopierbare UV-Leuchte 26 vorgesehen sein.

Fig. 3 zeigt die Vorrichtung 4 in einer ersten Schnittdarstellung. Hier ist eine Walzenbürsteneinheit 38 zu sehen, die mittig an der Unterseite 12 des Gehäuses 8 angeordnet ist. Sie ist um eine zweite Drehachse 40 rotierbar, die parallel zu dem Fußboden 6 verläuft. In der Zeichnungsebene links neben der Walzenbürsteneinheit 38 ist eine Abgabedüse 42 gezeigt, mit der ein Fluid aus einem Fluidtank 44 auf den Fußboden 6 aufgetragen bzw. aufgesprüht werden kann. In dem Fluidtank 44 könnte sich etwa eine Reinigungsmittel befinden, das zur Reinigung des Fußbodens 6 geeignet ist.

In der Zeichnungsebene rechts neben der Walzenbürsteneinheit 38 ist eine erste Saugdüse 46 vorgesehen, mit der Festpartikel und Flüssigkeit abgesaugt werden können. Sie steht mit einer Saugeinheit 45 in Fluidverbindung. Eine zweite Saugdüse 48 ist oberhalb der Walzenbürsteneinheit 38 vorgesehen und primär zum Absaugen von Festpartikeln vorgesehen. Sie kann ebenso mit der Saugeinheit 45 in Fluidverbindung stehen. Durch die zweite Saugdüse 48 aufgesaugte Stoffe gelangen in einen Abfallbehälter 50. Durch die erste Saugdüse 46 abgesagte Flüssigkeit gerät in einen Abwassertank 52, der in der Zeichnungsebene in Fig. 3 nicht zu sehen ist, jedoch in Fig. 4 dargestellt wird.

Zum Trennen von Flüssigkeit und Partikeln kann ein Abscheider eingesetzt werden. Alternativ dazu können die Saugdüsen 46 und 48 nacheinander mit der Saugeinheit 45 gekoppelt werden. Weiter alternativ ist eine zweite Saugeinheit denkbar, die ausschließlich zum Einsaugen von Flüssigkeit eingesetzt wird. In dem gezeigten Ausführungsbeispiel sind der Abwassertank 52 und der Fluidtank 44 an zwei einander entgegengesetzten Seiten des Gehäuses 8 angeordnet und umschließen dabei die Saugeinheit 45.

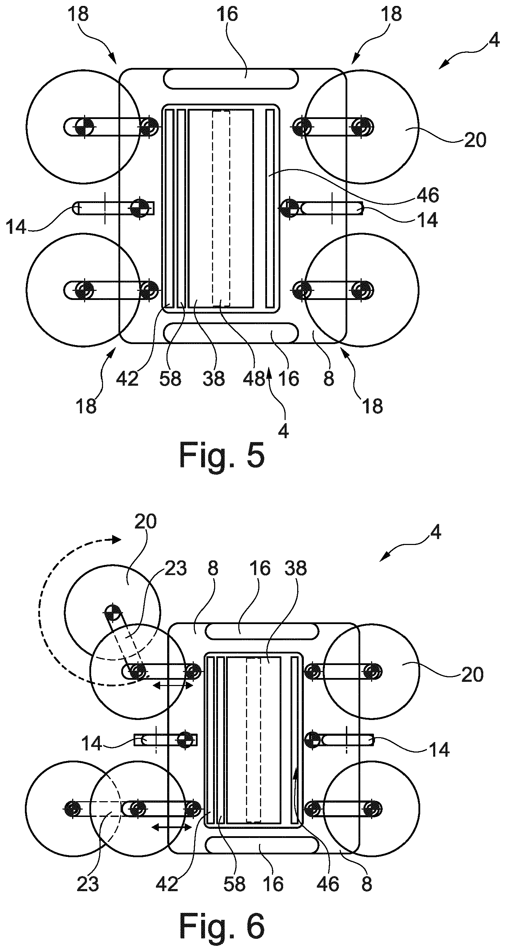

Eine Heizeinrichtung 54 einer Warmluftabgabeeinheit 56 ist vorgesehen, Luft zu erhitzen und durch eine Warmluftdüse 58 abzugeben. Diese kann parallel zu der Saugdüse 46 und der Abgabedüse 42 verlaufen und insbesondere zwischen der Abgabedüse 42 und der Walzenbürsteneinheit 38 angeordnet sein. Dies ist in den Fig. 5 und 6 dargestellt.

Weiterhin ist eine Steuereinheit 59 vorgesehen, die sich an der Oberseite 10 des Gehäuses 8 befindet. Diese kann mit den aktiven Komponenten der Vorrichtung 4 gekoppelt sein und dazu ausgebildet sein, durch Ansteuerung der Bürsteneinheiten 20 und 38, der Antriebseinheit 17, der Fluidzufuhreinheit und der Saugeinheit 45 den Fußboden 6 zu reinigen.

Die Vorrichtung 4 weist weiterhin eine Batterie 55 auf, die von außen zum Aufladen mit einer Stromquelle verbindbar ist. Die Batterie 55 versorgt die aktiven Komponenten mit elektrischer Leistung.

Wie weiter in Fig. 4 dargestellt wird, kann die Vorrichtung 4 zwei UV-Leuchten 26 aufweisen, die jeweils teleskopierbar und schwenkbar ausgebildet sind. Die Bestrahlungsleistung kann damit erhöht werden.

In Fig. 5 wird die Vorrichtung 4 von der Unterseite 12 gezeigt. Hier sind vier Eckbürsteneinheiten 20 vorgesehen, die zum Einnehmen der kompaktierten Stellung möglichst dicht an den Ecken 18 befinden. Wie Fig. 6 zeigt, werden die Eckbürsteneinheiten 20 zum Einnehmen der Betriebsstellung von dem Gehäuse 8 beabstandet, um eine bessere Erreichbarkeit von Ecken des Waschraums 2 zu ermöglichen. Hierzu können die vier Eckbürsteneinheiten 20 jeweils einen Schwenkarm 23 aufweisen, der eine translatorische Bewegung und ein Verschwenken erlaubt.

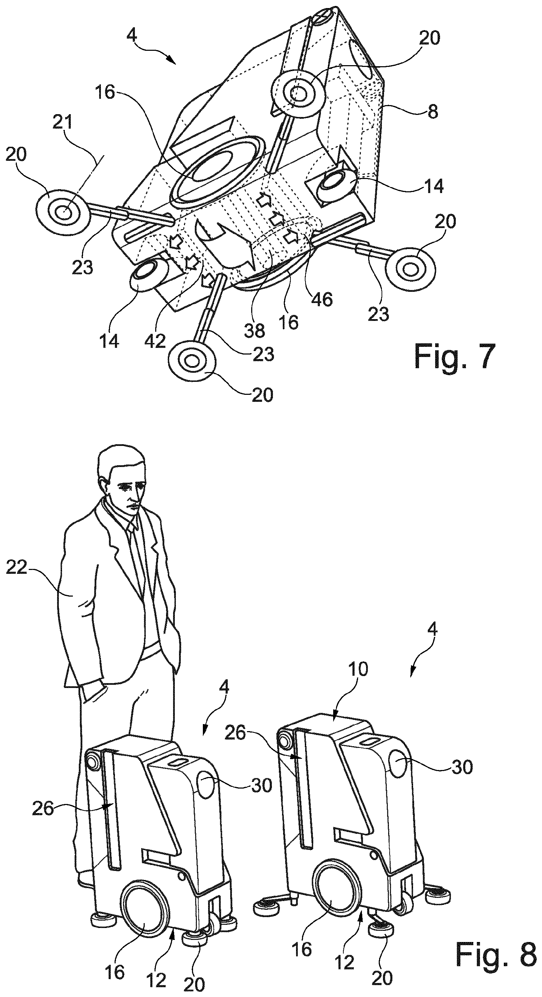

Fig. 7 zeigt die Vorrichtung 4 schräg von unten. Hier ist eine Drehrichtung der Walzenbürsteneinheit 38 erkennbar, durch die Schmutzpartikel aufgenommen werden. Gleichzeitig wird Fluid aus der Abgabedüse 42 abgegeben und Flüssigkeit durch die zweite Saugdüse 48 aufgesaugt.

In Fig. 8 ist die Vorrichtung 4 in einer kompaktierten Stellung (links) und der Betriebsstellung (rechts) gezeigt. Der Größenvergleich mit dem Benutzer 22 lässt erkennen, dass sich die Vorrichtung 4 leicht bewegen und verstauen lässt.

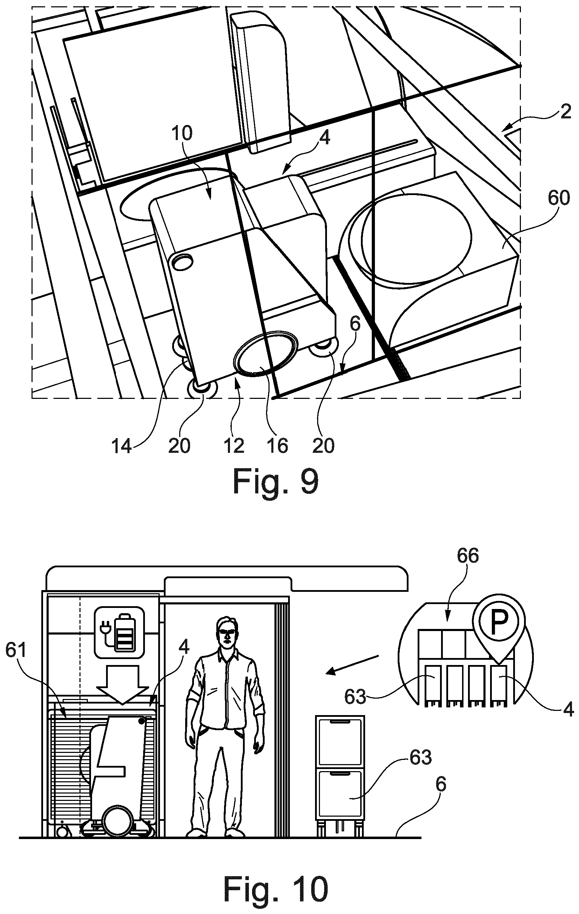

Fig. 9 zeigt die Vorrichtung 4 innerhalb des Waschraums 2 vor einer Toilette 60. Die Vorrichtung 4 ist dazu ausgebildet, in den Waschraum 2 hineingefahren zu werden, um dort selbstständig den Waschraum 2 zu reinigen.

Fig. 10 zeigt die Vorrichtung in einem Stellplatz 61, der beispielsweise für die Aufnahme von herkömmlichen Trolleys 63 ausgebildet ist. Hier kann eine Schnittstelle, beispielsweise über ein Kabel, zum Aufladen der Batterie 55 der Vorrichtung 4 vorgesehen sein. In der Zeichnungsebene rechts ist eine Bordküche 66 gezeigt, in der der Stellplatz 61 angeordnet sein kann, hier mit einem "P" markiert.

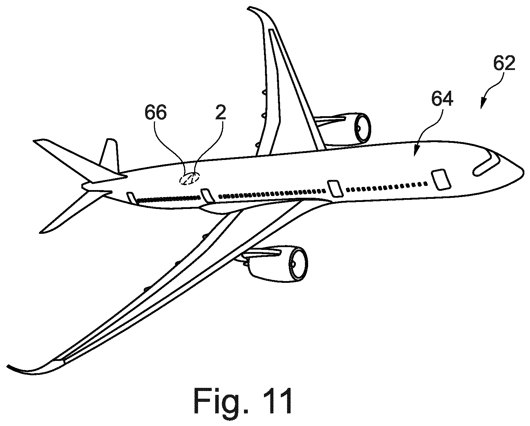

Schließlich zeigt Fig. 11 ein Flugzeug 62 mit einer Kabine 64, in der sich mindestens ein Waschraum 2 befindet. Dort kann eine Vorrichtung 4 eingesetzt werden. Bei Nichtgebrauch kann die Vorrichtung in eine bevorzugt benachbarte Bordküche 66 und dort in einen Stellplatz 61 gestellt werden.

Ergänzend ist darauf hinzuweisen, dass "umfassend" oder "aufweisend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Vorrichtung (4) zum Reinigen eines Raums in einer Fahrzeugkabine, aufweisend:
ein Gehäuse (8) mit einer Oberseite (10) und einer Unterseite (12),
mehrere an der Unterseite (12) angeordnete Räder (14, 16),
mindestens eine an der Unterseite (12) angeordnete Bürsteneinheit (20, 38),
mindestens eine Saugeinheit (45),
eine Fluidzufuhreinheit,
eine Antriebseinheit (17),
eine Steuereinheit (59), und
mindestens eine an der Oberseite (10) des Gehäuses (8) bewegbar angeordnete UV-Leuchte (26) zum Sterilisieren von Oberflächen,
wobei die Antriebseinheit (17) mit mindestens einem der Räder (14, 16) zum Bewegen der Vorrichtung (4) auf einem Fußboden (6) des Raums (2) gekoppelt ist,
wobei die Fluidzufuhreinheit mindestens eine Abgabedüse (42) aufweist, die auf den Fußboden (6) und/oder zumindest eine der mindestens einen Bürsteneinheit (20, 38) gerichtet ist,
wobei die mindestens eine Saugeinheit (45) mindestens eine Saugdüse (46, 48) aufweist, die auf den Fußboden (6) und/oder zumindest eine der mindestens einen Bürsteneinheit (20, 38) gerichtet ist und dazu ausgebildet ist, Feststoffe und/oder Flüssigkeiten aufzusaugen,
wobei die mindestens eine Bürsteneinheit (20, 38) dazu ausgebildet ist, durch eine rotierende Bewegung den Fußboden (6) zu reinigen, und
wobei die Steuereinheit (59) mit der Antriebseinheit (17), der mindestens einen Saugeinheit, und der Fluidzufuhreinheit gekoppelt ist und dazu ausgebildet ist, die Vorrichtung (4) durch Ansteuerung der Antriebseinheit (17) auf dem Fußboden (6) zu verfahren und dabei durch Ansteuerung der mindestens Bürsteneinheit (20, 38), der Fluidzufuhreinheit und der Saugeinheit den Fußboden (6) zu reinigen.

2. Vorrichtung (4) nach Anspruch 1,
wobei die mindestens eine UV-Leuchte (26) teleskopierbar und/oder schwenkbar angeordnet ist, sodass eine Oberfläche eines Waschtischs und/oder ein Türgriff damit beleuchtbar ist.

3. Vorrichtung (4) nach einem der vorhergehenden Ansprüche,
ferner aufweisend eine Luftreinigungseinheit, die dazu ausgebildet ist, Luft aus dem zu reinigenden Raum einzusaugen und gereinigt wieder abzugeben.

4. Vorrichtung (4) nach Anspruch 3,
wobei die Luftreinigungseinheit (30) mindestens einen Luftkanal (32) aufweist, durch den die eingesaugte Luft strömt und mittels UV-Strahlen gereinigt wird.

5. Vorrichtung (4) nach einem der vorhergehenden Ansprüche,
wobei die mindestens eine Bürsteneinheit (20, 38) mehrere Eckbürsteneinheiten (20) aufweist, die an Ecken des Gehäuses (8) angeordnet sind und jeweils um eine erste Drehachse (21) rotierbar sind, die zu dem Fußboden (6) im Wesentlichen vertikal verläuft.

6. Vorrichtung (4) nach Anspruch 5,
wobei mindestens eine Eckbürsteneinheit (20) relativ zu dem Gehäuse (8) bewegbar ist, sodass sie einen variablen Abstand und/oder eine variable Ausrichtung zu dem Gehäuse (8) einnehmen kann.

7. Vorrichtung (4) nach Anspruch 5 oder 6,
wobei die mindestens eine Bürsteneinheit (20, 38) vier Eckbürsteneinheiten (20) umfasst, die an vier Ecken (18) der Unterseite (12) des Gehäuses (8) angeordnet sind.

8. Vorrichtung (4) nach einem der vorhergehenden Ansprüche,
wobei die mindestens eine Bürsteneinheit (20, 38) eine Walzenbürsteneinheit (38) aufweist, die um eine zweite Drehachse (40) rotierbar ist, die im Wesentlichen parallel zu dem Fußboden (6) ausgerichtet ist.

9. Vorrichtung (4) nach Anspruch 8,
wobei die Walzenbürsteneinheit (38) zwischen der mindestens einen Abgabedüse (42) und der mindestens einen Saugdüse (46, 48) angeordnet ist.

10. Vorrichtung (4) nach Anspruch 8 oder 9,
wobei die Walzenbürsteneinheit (38) mittig an der Unterseite (12) angeordnet ist.

11. Vorrichtung (4) nach einem der vorhergehenden Ansprüche,
ferner aufweisend eine Warmluftabgabeeinheit (56) mit mindestens einer Warmluftdüse (58), die an der Unterseite (12) angeordnet und auf den Fußboden (6) gerichtet ist, um warme Luft zum Trocknen des Fußbodens (6) abzugeben.

12. Vorrichtung (4) nach einem der vorhergehenden Ansprüche,
wobei das Gehäuse (8) eine Länge von etwa 0,4 m, eine Breite von etwa 0,3 m und eine Höhe von etwa 1 m aufweist, die im Wesentlichen der Größe eines Half-Size-Trolley für Luftfahrzeugkabinen entspricht.

13. Luftfahrzeug (62), aufweisend einen Raum (2) mit mehreren Wandungen und einem Fußboden (6), sowie eine Vorrichtung (4) nach einem der vorhergehenden Ansprüche.

14. Luftfahrzeug (66) nach Anspruch 13,
wobei der Raum (2) ein Waschraum (2) mit einer Toilette (60) und/oder einem Waschtisch ist, und
wobei das Luftfahrzeug (66) einen Trolley-Stellplatz (61) aufweist, in dem die Vorrichtung (4) abstellbar ist.

## Claims

1. Device (4) for cleaning a room in a vehicle cabin, having:
a housing (8) with a top side (10) and a bottom side (12),
a plurality of wheels (14, 16) arranged on the bottom side (12),
at least one brush unit (20, 38) arranged on the bottom side (12),
at least one suction unit (45),
a fluid supply unit,
a drive unit (17),
a control unit (59), and
at least one UV lamp (26), which is movably arranged on the top side (10) of the housing (8), for sterilizing surfaces,
wherein the drive unit (17) is coupled to at least one of the wheels (14, 16) for moving the device (4) on a floor (6) of the room (2),
wherein the fluid supply unit has at least one discharge nozzle (42) which is directed towards the floor (6) and/or at least one of the at least one brush unit (20, 38),
wherein the at least one suction unit (45) has at least one suction nozzle (46, 48) which is directed towards the floor (6) and/or at least one of the at least one brush unit (20, 38) and is designed to suck up solids and/or liquids,
wherein the at least one brush unit (20, 38) is designed to clean the floor (6) by way of a rotating movement, and
wherein the control unit (59) is coupled to the drive unit (17), the at least one suction unit and the fluid supply unit and is designed to move the device (4) on the floor (6) by actuating the drive unit (17) and, in the process, clean the floor (6) by actuating the at least brush unit (20, 38), the fluid supply unit and the suction unit.

2. Device (4) according to Claim 1,
wherein the at least one UV lamp (26) is arranged so as to be telescopable and/or pivotable, such that a surface of a washbasin and/or a door handle are able to be illuminated thereby.

3. Device (4) according to one of the preceding claims, further having an air cleaning unit which is designed to suck in air from the room that is to be cleaned and to discharge it again in a cleaned state.

4. Device (4) according to Claim 3,
wherein the air cleaning unit (30) has at least one air duct (32) through which the sucked-in air flows and is cleaned by means of UV rays.

5. Device (4) according to one of the preceding claims, wherein the at least one brush unit (20, 38) has a plurality of corner brush units (20) which are arranged at corners of the housing (8) and are each able to be rotated about a first axis of rotation (21) which runs substantially vertically with respect to the floor (6).

6. Device (4) according to Claim 5,
wherein at least one corner brush unit (20) is able to be moved relative to the housing (8) such that it can take up a variable distance and/or a variable alignment with respect to the housing (8).

7. Device (4) according to Claim 5 or 6,
wherein the at least one brush unit (20, 38) comprises four corner brush units (20) which are arranged at four corners (18) of the bottom side (12) of the housing (8).

8. Device (4) according to one of the preceding claims, wherein the at least one brush unit (20, 38) has a roller brush unit (38) which is able to be rotated about a second axis of rotation (40) which is oriented substantially parallel to the floor (6).

9. Device (4) according to Claim 8,
wherein the roller brush unit (38) is arranged between the at least one discharge nozzle (42) and the at least one suction nozzle (46, 48).

10. Device (4) according to Claim 8 or 9,
wherein the roller brush unit (38) is arranged centrally on the bottom side (12).

11. Device (4) according to one of the preceding claims, further having a warm air discharge unit (56) with at least one warm air nozzle (58) which is arranged on the bottom side (12) and is directed towards the floor (6) in order to discharge warm air for drying the floor (6).

12. Device (4) according to one of the preceding claims, wherein the housing (8) has a length of about 0.4 m, a width of about 0.3 m and a height of about 1 m, which substantially corresponds to the size of a half-size trolley for aircraft cabins.

13. Aircraft (62) having a room (2) with a plurality of walls and a floor (6), and a device (4) according to one of the preceding claims.

14. Aircraft (66) according to Claim 13,
wherein the room (2) is a washroom (2) with a toilet (60) and/or a washbasin, and
wherein the aircraft (66) has a trolley parking space (61) in which the device (4) is able to be parked.

## Revendications

1. Dispositif (4) de nettoyage d'un espace dans une cabine de véhicule, comportant :
un boîtier (8) présentant un côté supérieur (10) et un côté inférieur (12),
plusieurs roues (14, 16) disposées sur le côté inférieur (12),
au moins une unité de brossage (20, 38) disposée sur le côté inférieur (12),
au moins une unité d'aspiration (45),
une unité d'arrivée de fluide,
une unité d'entraînement (17),
une unité de commande (59), et
au moins une lampe UV (26) pour stériliser des surfaces, disposée de manière mobile sur le côté supérieur (10) du boîtier (8),
l'unité d'entraînement (17) étant couplée à au moins une des roues (14, 16) pour déplacer le dispositif (4) sur un sol (6) de l'espace (2),
l'unité d'arrivée de fluide comportant au moins une buse de distribution (42) qui est dirigée vers le sol (6) et/ou vers au moins une de l'au moins une unité de brossage (20, 38),
l'au moins une unité d'aspiration (45) comportant au moins une buse d'aspiration (46, 48), qui est dirigée vers le sol (6) et/ou au moins vers une de l'au moins une unité de brossage (20, 38) et étant conçue pour aspirer des matières solides et/ou des liquides,
l'au moins une unité de brossage (20, 38) étant conçue pour nettoyer le sol (6) par un mouvement de rotation, et
l'unité de commande (59) étant couplée à l'unité d'entraînement (17), à l'au moins une unité d'aspiration et à l'unité d'arrivée de fluide et étant conçue pour déplacer le dispositif (4) par commande de l'unité d'entraînement (17) sur le sol (6) et ainsi nettoyer le sol (6) par commande de l'au moins une unité de brossage (20, 38), de l'unité d'arrivée de fluide et de l'unité d'aspiration.

2. Dispositif (4) selon la revendication 1,
l'au moins une lampe UV (26) étant disposée de manière télescopique et/ou de manière à pouvoir pivoter, de sorte qu'une surface d'un plan de toilette et/ou une poignée de porte peuvent être éclairées avec celle-ci.

3. Dispositif (4) selon l'une des revendications précédentes,
comportant en outre une unité de purification d'air, qui est conçue pour aspirer de l'air provenant de l'espace à nettoyer et pour le rejeter de nouveau une fois purifié.

4. Dispositif (4) selon la revendication 3,
l'unité de purification d'air (30) comportant au moins un conduit d'air (32), à travers lequel l'air aspiré s'écoule et est purifié au moyen de rayons UV.

5. Dispositif (4) selon l'une des revendications précédentes,
l'au moins une unité de brossage (20, 38) comportant plusieurs unités de brossage d'angle (20), qui sont disposées aux coins du boîtier (8) et peuvent tourner chacune autour d'un premier axe de rotation (21) qui s'étend sensiblement verticalement par rapport au sol (6).

6. Dispositif (4) selon la revendication 5,
au moins une unité de brossage d'angle (20) pouvant être déplacée par rapport au boîtier (8) de sorte qu'elle peut prendre une distance et/ou une orientation variables par rapport au boîtier (8).

7. Dispositif (4) selon la revendication 5 ou 6,
l'au moins une unité de brossage (20, 38) comprenant quatre unités de brossage d'angle (20), qui sont disposées aux quatre coins (18) du côté inférieur (12) du boîtier (8).

8. Dispositif (4) selon l'une des revendications précédentes,
l'au moins une unité de brossage (20, 38) comportant une unité de brossage à rouleau (38), qui peut tourner autour d'un deuxième axe de rotation (40), qui est orienté sensiblement parallèlement au sol (6).

9. Dispositif (4) selon la revendication 8,
l'unité de brossage à rouleau (38) étant disposée entre l'au moins une buse de distribution (42) et l'au moins une buse d'aspiration (46, 48).

10. Dispositif (4) selon la revendication 8 ou 9, l'unité de brossage à rouleau (38) étant disposée au centre sur le côté inférieur (12).

11. Dispositif (4) selon l'une des revendications précédentes,
comportant en outre une unité de distribution d'air chaud (56) comportant au moins une buse d'air chaud (58) qui est disposée sur le côté inférieur (12) et est dirigée vers le sol (6) pour distribuer de l'air chaud pour sécher le sol (6).

12. Dispositif (4) selon l'une des revendications précédentes,
le boîtier (8) présentant une longueur d'environ 0,4 m, une largeur d'environ 0,3 m et une hauteur d'environ 1 m, ce qui correspond sensiblement à la taille d'un chariot demi-format pour des cabines d'aéronef.

13. Aéronef (62), comportant un espace (2) présentant plusieurs parois et un sol (6), ainsi qu'un dispositif (4) selon l'une des revendications précédentes.

14. Aéronef (66) selon la revendication 13,
l'espace (2) étant un cabinet de toilette (2) comportant des toilettes (60) et/ou un plan de toilette, et
l'aéronef (66) comportant un emplacement pour chariot (61), dans lequel le dispositif (4) peut être placé.
